# EUROPEAN PATENT APPLICATION

(11) **EP 1 288 291 A1**
(43) Date of publication of application: **05.03.2003**
(21) Application number: 01936857.0
(22) Date of filing: 05.06.2001
(51) Int. Cl.: C12N 5/02, A61K 35/14, A61P 35/00

(54) **METHOD OF AMPLIFYING NATURAL KILLER T CELLS**

(30) Priority: 06.06.2000 JP 2000169430
(71) Applicant: KIRIN BEER KABUSHIKI KAISHA, Tokyo 104-8288 (JP)
(72) Inventor: WAKASUGI, Hiro, National Cancer Center, Chuo-ku, Tokyo 104-0045 (JP)
(74) Representative: HOFFMANN - EITLE
(86) International application number: JP0104746
(87) International publication number: WO01094553

(57) **Abstract**

Human Vα24⁺ natural killer T cells are expanded by culturing a mononuclear cell fraction obtainable from human peripheral blood in which hemopoietic stem cells are mobilized by granulocyte colony-stimulating factor, in the presence of a cytokine, such as interleukin 2, effecting proliferation and/or activation of lymphocytes and α-glycosylceramide. A cell fraction comprising human Vα24⁺ natural killer T cells expanded by the method, is useful as a cancer-treating agent.

## Description

### Technical Field

The present invention relates to a method for expanding human Vα24⁺ natural killer T (NKT) cells, and uses of human Vα24⁺ NKT cells obtained by the method and a fraction comprising the human Vα24⁺ NKT cells.

### Background Art

NKT cells are an exceptional subset of mature lymphocytes that bear both NK and T cell receptors (Annu. Rev. Immunol., 15, 535-562, 1997; J. Exp. Med., 182, 633-638, 1995). Murine NKT cells express NK1.1 and TCRαβ receptors and are especially dense in the bone marrow (J. Immunol., 145, 3209-3215, 1990) and liver (J. Exp. Med., 180, 699-704, 1994). The cells express a very limited TCR repertoire (J. Exp. Med., 180, 1097-1106, 1994; Int. Immunol., 7, 1157-1161, 1995), including an invariant α-chain. These suggest that the ligand for NKT cells is non-polymorphic, and a non-classical MHC class I molecule that appear to present a specific antigen processed via TAP (transporter associated with antigen processing)-independent pathway. A recent study determined that one the MHC class Ib molecules, CD1d, is a ligand for NKT cells. Human T cells expressing the invariant Vα24JαQ TCR with canonical rearrangements without N regions have recently been reported to be analogous to the murine Vα14-Jα281⁺ NKT cells (J. Exp. Med., 180, 1097-1106, 1994). Moreover, murine Vα14-Jα281⁺ cells and human Vα24JαQ TCR⁺ NKT cells have been shown to proliferate upon stimulation with CD1d antigen presenting cells (APCs) pretreated with α-galactosylceramide (α-GalCer). Activated NKT cells reportedly display an NK-like perforin-dependent cytotoxicity against various tumor cell lines (Cancer Res., 59, 5102-5105, 1999) and inhibit tumor metastasis in certain experimental animal models (Pro. Natl. Acad. Sci. USA, 95, 5690-5693, 1998).

In the 1980's, numerous immunotherapy clinical trials were carried out, including those investigating LAK (lymphokine-activated killer) cell therapy and TIL (tumor-infiltrating lymphocytes) therapy, with or without cytokine injection. However, the findings of these clinical trials demonstrated that these cell-therapies fall short of expectations in terms of their clinical effect, with not obvious clinical benefit being detected. Significant progress has been made in the 1990's in identifying the molecular components of the immune response to human cancer. Some different immunotherapeutic approaches, such as dendritic cell (DC) therapy and cytotoxic T lymphocyte (CTL) therapy are currently being explored in clinical trails.

As mentioned above, NKT cells, distinct from other lymphoid cells including T cells, B cells, and NK cells, are characterized by coexpression of the NK receptor and an invariant T cell receptor. It is known that this novel lineage lymphocyte can produce large amounts of interleukin 4 (IL-4) and interferon γ (IFN-γ) and shows strong tumor-killing activity. Therefore, the role of NKT cells is important in cancer immune-therapy, and these cells are expected to represent a novel immunotherapy using the ability of these cells. Some studies have investigated *ex vivo* expansion of Vα24⁺ NKT cells (Cancer Res., 59, 5102-5105, 1999; Immunology, 99, 229-234, 2000). However, it is difficult to obtain a sufficient number of the cells to realize immunotherapy, even from cord blood.

### Disclosure of Invention

An object of the present invention is to provide a method for producing a sufficient number of Vα24⁺ NKT cells. Also, an object of the present invention is to provide Vα24⁺ NKT cells and a fraction comprising Vα24⁺ NKT cells which are suitable for use in a cell therapy which is a therapy for a disease by using cells.

The present inventors have found that a method for obtaining a sufficient number of Vα24⁺ NKT cells, by using α-GlyCer, from a mononuclear cell fraction obtainable from human peripheral blood in which hemopoietic stem cells are mobilized by granulocyte colony-stimulating factor (G-CSF) (G-PBSCs).

The mononuclear cell fraction includes DCs and T cells, monocytes, B cells, NK cells, NKT cells, granulocytes and the like. When α-GlyCer affects the fraction, α-GlyCer is presented on CD1d molecule of DC, and Vα24⁺ NKT cells, NK cells and the like proliferate due to secretion of cytokines. When the mononuclear cell fraction is obtained from peripheral blood in which hemopoietic stem cells are mobilized by G-CSF, more efficient proliferation of NKT cells occurs and more potent immune response of the mononuclear cell fraction is caused. Also, expansion of NKT cells caused by culturing a mononuclear cell fraction obtainable from peripheral blood in which hemopoietic stem cells are mobilized by G-CSF (G-PBSCs), in the presence of IL-2 and α-GlyCer, continues longer compared with a mononuclear cell fraction obtainable from peripheral blood without mobilization by G-CSF (PBMCs).

The present invention has been achieved based on the findings, and provides expansion methods and production methods (including cells and cell fractions obtainable by the methods) and agents for treating cancer as mentioned below:
(1) A method for expanding human Vα24⁺ NKT cells, comprising culturing a mononuclear cell fraction obtainable from human peripheral blood in which hemopoietic stem cells are mobilized by G-CSF (G-PBSCs), in the presence of a cytokine effecting proliferation and/or activation of lymphocytes and α-GlyCer.
(2) A method according to (1), wherein the cytokine effecting proliferation and/or activation of lymphocytes comprises IL-2.
(3) A method for producing human Vα24⁺ NKT cells, comprising culturing a mononuclear cell fraction obtainable from human peripheral blood in which hemopoietic stem cells are mobilized by G-CSF (G-PBSCs), in the presence of a cytokine effecting proliferation and/or activation of lymphocytes and α-GlyCer, and isolating human Vα24⁺ natural killer T cells from the cultured cells.
(4) A method according to (3), wherein the cytokine effecting proliferation and/or activation of lymphocytes comprises IL-2.
(5) A method for producing a cell fraction comprising human Vα24⁺ NKT cells, comprising culturing a mononuclear cell fraction obtainable from human peripheral blood in which hemopoietic stem cells are mobilized by G-CSF (G-PBSCs), in the presence of a cytokine effecting proliferation and/or activation of lymphocytes and α-GlyCer.
(6) A method according to (5), further comprising culturing cells obtained by the culture in the presence of IL-2 and α-GlyCer, in the presence of a tumor antigen.
(7) A method according to (5) or (6), wherein the cytokine effecting proliferation and/or activation of lymphocytes comprises IL-2.
(8) An agent for treating cancer, comprising, as an active ingredient, human Vα24⁺ NKT cells obtained by the method as defined in (3) or (4).
(9) An agent for treating cancer, comprising, as an active ingredient, a fraction comprising human Vα24⁺ NKT cells obtained by the method as defined in any one of (5) to (7).

Also, the present invention provides a method for treating cancer, comprising administering, to a subject in need of cancer treatment, an effective amount of human Vα24⁺ NKT cells obtained by the method as defined in (3) or (4) or a fraction comprising human Vα24⁺ NKT cells obtained by the method as defined in any one of (5) to (7). Further, the present invention provides a use of human Vα24⁺ NKT cells obtained by the method as defined in (3) or (4) or a fraction comprising human Vα24⁺ NKT cells obtained by the method as defined in any one of (5) to (7) in manufacture of an agent for treating cancer.

### Brief Description of Drawings

Fig. 1 shows representative flow cytometric profiles of Vα24⁺ NKT cells from PBMCs and G-PBSCs expanded in response to IL-2 and α-GalCer. Left: after culture, middle: with α-GalCer, right: without α-GalCer.
Fig. 2 shows numbers of Vα24⁺ NKT cells before and after the culture when PBMCs or G-PBSCs were cultured in the presence of α-GalCer and 100 U/ml IL-2 for 12 days.
Fig. 3 shows FACS profiles of α-GalCer-activated Vα24⁺ NKT cells. PBMCs or G-PBSCs were cultured in the presence of α-GalCer and 100 U/ml IL-2 for 12 days and the phenotype of the cultured cells were analyzed.
Fig. 4 shows antitumor cytotoxic activity of α-GalCer-activated Vα24⁺ NKT cells. The cytotoxicity was measured by ^{5l}Cr release assay against Molt-4 T lymphoma (1 x 10⁴; left panel) and K562 myelogeous leukemia (1 x 10⁴; right panel).
Fig. 5 shows day-by-day changes of numbers of Vα24⁺ NKT cells from PBMCs and G-PBSCs expanded in response to IL-2 and α-GalCer.

### Best Mode for Carrying Out the Invention

The present invention is described in details.

The expansion methods and production methods according to the present invention are characterized by culturing a mononuclear cell fraction obtainable from human peripheral blood in which hemopoietic stem cells are mobilized by G-CSF (G-PBSCs), in the presence of a cytokine effecting proliferation and/or activation of lymphocytes and α-GlyCer.

G-PBSCs may be prepared by, for example, the following method.

When G-CSF is subcutaneously administered to cancer patients at a dose of 2 to 16 µg/kg/day for 5 to 10 days, numbers of CD34⁺ cells and colony-forming unit-granulocyte macrophage (CFU-GM) in peripheral blood, which are indexes of hemopoietic stem cells, increase and each show a highest value around from day 5 to day 6. Therefore, 4 days to 12 days after start of G-CSF administration, a cell fraction containing peripheral blood stem cells (crude G-PBSCs) is collected by apheresis. A mononuclear cell fraction (G-PBSC) is prepared therefrom by further using density gradient centrifugation and the like. G-PBSCs may be also prepared from healthy persons by the similar method.

A fraction of mononuclear cells obtained from peripheral blood mainly contains mononuclear cells and granulocytes such as monocytes and lymphocytes (T cells, B cells, NK cells, NKT cells), and erythrocytes and platelets are removed therefrom.

Generally, peripheral blood from healthy persons contains 0.02% of hemopoietic stem cells. Compared with this, in peripheral blood from healthy persons in which mobilization is effected by administration of G-CSF, it increases to about from 0.5 to 1%, and in peripheral blood from cancer persons in which mobilization is effected by administration of G-CSF after chemotherapy, it increases about from 2 to 7%. Consequently, the mononuclear cell fraction obtained from peripheral blood in which hemopoietic stem cells are mobilized by G-CSF (G-PBSCs) contains more hemopoietic stem cells compared with the mononuclear cell fraction obtained from peripheral blood in which the hemopoietic cells are not mobilized by G-CSF.

Culture may be performed under the usual culture conditions for PBSCs provided that the cytokine effecting proliferation and/or activation of lymphocytes and α-GlyCer are added to a culture medium. The added amounts of the cytokine effecting proliferation and/or activation of lymphocytes and α-GlyCer and a culture period may be those sufficient to expand Vα24⁺ NKT cells. Usually, although it may vary depending on kinds of cytokines effecting proliferation and/or activation of lymphocytes, a cytokine concentration is 50 to 200 U/ml, an α-GlyCer concentration is usually 10 to 200 ng/ml, and a culture period is 2 to 40 days. Expansion of Vα24⁺ NKT cells may be determined by analysis with flow cytometry of fluorescent-antibody-stained cells.

α-GlyCer is a sphingoglycolipid in which a sugar chain of galactose, glucose and the like is bound to ceramid by α-linkage. Examples thereof include those disclosed by WO 93/05055 (published March 18, 1993), WO 94/02168 (published February 3, 1994), WO 94/09020 (published April 28, 1994), WO 94/24142 (published October 27, 1994) and WO 98/44928 (published October 15, 1998). In particular, (2S,3S,4R)-1-*O*-(α-D-galactopyranosyl)-*N*-hexacosanoyl-2-amino-1,3,4-octadecanetriol) is preferable.

Examples of the cytokine effecting proliferation and/or activation of lymphocytes include IL-2, IL-7, IL-15, IL-12 and IL-18. The cytokine may be used alone or two or more cytokines may be used in combination. Preferably, the cytokine comprises IL-2, that is, IL-2 or a combination of IL-2 with another cytokine.

As a medium used for the culture, it may be mentioned RPMI-1640 containing 2 mM L-glutamine, 1% pyruvate, 2% bicarbonate, 100 U/ml penicillin, and 100 U/ml streptomycin supplemented with 10% fetal calf serum (FCS).

By culturing the mononuclear cell fraction in which hemopoietic stem cells are mobilized by G-CSF as described above, the number of Vα24⁺ NKT cells remarkably increases compared by the case of culturing PBMCs without mobilization by G-CSF in the same manner.

In the previous clinical trials using LAK cells and TILs, 10 x 10¹⁰⁻¹¹ of effector cells were administered to patients. In contrast, according to the present invention, for example, on average, 8-40 x 10⁹ mononuclear cells were collected from the peripheral blood where mobilization by G-CSF is performed, and after 12 days of culture of them with IL-2 and α-GlyCer 1 x 10⁹⁻¹⁰ of autologous Vα24⁺ NKT cells may be obtained. The quantity of is equivalent to previous clinical studies, and sufficient clinically for use in immunotherapy.

According to the expansion method of the present invention, therefore, Vα24⁺ NKT cells of which ability to kill various kinds of tumors is known can be efficiently proliferated to an practical extent. Also, the cell fraction containing Vα24⁺ NKT cells, obtained by the production method of the present invention causes potent immune response.

In the production method of the present invention, after culturing cells obtained by the culture in the presence of the cytokine effecting proliferation and/or activation of lymphocytes and α-GlyCer, the cell are preferably cultured in the presence of a tumor antigen.

The culture in the presence of a tumor antigen may be performed under usual conditions for PBSCs provided that the tumor antigen is added to a culture medium. The added amount of the tumor antigen and a culture period may be those so sufficient that DCs contained in the cell fraction obtained by culture in the presence of the cytokines effecting proliferation and/or activation of lymphocytes and α-GlyCer, induce CTL. Usually, although it may vary depending on kinds of tumor antigens, a tumor antigen concentration is 10 to 10000 µg/ml, and a culture period is 2 to 14 days. Induction of CTL may be determined by the precursor frequency assay (Sharrock CE et al: Immunology Toda 11:281-286, 1990), the ELISPOT method (Pass H et al: Cancer J Sci Am 4:316-323, 1998), the tetramer method (Romero P et al, J Exp Med 188:1641-1650, 1998) and the like.

By the culture in the presence of the tumor antigen, CTL is induced in the cell fraction, and, therefore, the cell fraction may also cause immune response specific to a tumor antigen.

Isolation of Vα24⁺ NKT cells from cultured cells may be performed by usual methods such as the magnetic bead method.

The agent for treating cancer of the present invention comprises, as an active ingredient, human Vα24⁺ NKT cells or a fraction comprising human Vα24⁺ NKT cells obtained by the method of the present invention.

The human Vα24⁺ NKT cells obtained by the production method of the present invention are expected to potently cause the tumor antigen-specific immune response and eliminate tumor cells.

In the cell fraction containing human Vα24⁺ NKT cells, obtained by the production method of the present invention, the expansion of NKT cells continues longer compared with a cell fraction obtained by culturing a mononuclear cell fraction PBMCs without mobilization by G-CSF, in the presence of the cytokine effecting proliferation and/or activation of lymphocytes and α-GlyCer.

Furthermore, the cell fraction containing human Vα24⁺ NKT cells, obtained by further performing the culture in the presence of the tumor antigen can cause tumor antigen-specific immune response by induction of CTL by DCs in the fraction. That is, it potently causes both of the non-tumor antigen-specific immune response by expansion of human Vα24⁺ NKT cells and the tumor antigen-specific immune response. It is reported that the expression level of the major histocompatibility antigen (MHC) varies depending on portions of a tumor tissue (i.e., tumor cells). By causing both of immune responses, attack of tumor antigen-specific CTL against MHC-expressing tumor cells and attack of non-tumor antigen specific NKT cells against tumor cells expressing MHC at a low level or not expressing MHC are expected. Therefore, the cell fraction of this embodiment is advantageous for use in cancer treatment.

Therefore, human Vα24⁺ NKT cells or the cell fraction containing the human Vα24⁺ NKT cells, obtained by the production method of the present invention, are advantageous as an active ingredient of a cancer-treating agent.

Human Vα24⁺ NKT cells obtained by the production method of the present invention may be used for cell therapy such as cancer treatment, in combination with DCs separately isolated another cell group and activated by treatment with a tumor antigen. The activated DCs may be obtained by treating monocytes contained in PBSCs with GM-CSF and IL-4 or IL-3, or treating CD34-positive hemopoietic stem cells with GM-CSF or IFNα.

As an administration route of the agent for treating cancer of the present invention, intravenous, subcutaneous, intradermal, intralymphonodus administrations may be mentioned. As a form of the agent, a suspension in physiological saline or a Linger solution and the like may be mentioned. The administration amount may be different depending on the administration route, and 0.1 ml to 5 ml, if subcutaneously, or upto 300 ml, if intravenously, may be administered. The administration amount in terms of Vα24⁺ NKT cells is usually 10⁵ to 10¹⁰ per body surface (m²). As a disease to be treated, multiple myeloma, lymphoma, leukemia, breast cancer, colon cancer, lung cancer, prostate cancer, melanoma, kidney cancer, liver cancer, pancreas cancer and the like may be mentioned. Also, it is applicable to viral diseases such as CML and HIV.

According to the present invention, a large amount of autologous Vα24⁺ NKT cells may be produced *ex vivo* by using α-GlyCer. The cell number is high sufficiently to use in the clinical area. Furthermore, cultured Vα24⁺ NKT cells shows potent tumor-killing activity against a tumor irrespective of MHC expression. In the present invention, G-PBSCs shows very effective *in vitro* expansion of human Vα24⁺ NKT cells by stimulation of α-GlyCer compared with PBMCs. G-PBSCs are expanded to the same degree in either case that G-PBSCs are from healthy persons or cancer patients.

### Example

The present invention is described below in details with reference to Examples.

### Example 1. Expansion of human Vα24⁺ NKT cells

### (1) Preparation of PBMCs and preparation of G-PBSCs

Buffy coat (crude PBMCs), prepared from 400 ml of whole blood drawn from a healthy person, was supplied by the Japanese Red Cross Blood Center. A cell fraction containing PBSCs (crude G-PBSCs) was obtained from patients undergone chemotherapy followed by a dose of 100 to 250 µg/body/day G-CSF subcutaneously for 6 to 10 days (Table 1) by performing apheresis between 2 and 24 hours after the last injection of G-CSF with COBE Spectra™ Cell Separator (LAKE WOOD, CO USA 80215) (Hematopoietic Stem Cells: Biology and Therapeutic Applications. Levit DJ, Mertelsmann R(eds), Marcel Dekker, Inc., New York, 1995, 611-630).

From the obtained crude PBMCs and crude G-PBSCs, mononuclear cell fractions (referred to as "PBMCs" and "G-PBSCs", respectively) were prepared using Lymphosepal density-gradient medium (Immuno-Biological Laboratories Gunma, Japan).

**Table 1.**

| Patient Characteristics | | | | | |
|---|---|---|---|---|---|
| No. | Age | Sex | Disease | Chemotherapy | G-CSF |
| 6 | 9 | M | Ewing's sarcoma | VCR/CPM/ADM | Lenograstim 100 µl |
| 7 | 38 | F | Breast Ca. | ADM/DTX | Lenograstim 100 µl |
| 8 | 43 | F | Ovarian Ca. | Taxol/CDDP | Lenograstim 250 µl |
| 9 | 32 | M | NSGCT | VP16/CDDP | Lenograstim 100 µl |
| 10 | 27 | F | Breast Ca. | ADM/DTX | Lenograstim 100 µl |
| NSGCT: non-seminomatous germ cell tumors | | | | | |

### Activation and expansion of Vα24⁺ NKT cells in vitro

PBMCs and G-PBSCs obtained in (1) were cultured with RPMI-1640 supplemented with 2 mM L-glutamine, 1% pyruvate, 2% bicarbonate, 100 U/ml penicillin, 100 U/ml streptomycin (GIBCO BRL) in the presence of 100 U/ml IL-2 and 100 ng/ml of α-GlyCer. Cells were incubated in a water-vapor-saturated atmosphere containing 5% CO₂ gas at 37°C. The cell number and characteristics of these cultured cells were examined on day 0 (d0) and day 12 (dl2). As α-GlyCer, (2S,3S,4R)-1-O-(α-D-galactopyranosyl)-N-hexacosanoyl-2-amino-1,3,4-octadecanetriol; referred to as "α-GalCer") prepared by Kirin Beer Kabushiki Kaisha was used.

Flow cytometry of the cultured cells was performed using a FACScan cytometer (Becton Dickinson, Sunyvale, CA) as follows. Fluorescein isothiocynate (FITC)-conjugated anti-Vα24 (C15), phycoerythrin (PE)-conjugated anti-Vβ11 (C21), PE-conjugated anti-CD3, PE-conjugated anti-CD56, PE-conjugated anti-DC83 and PE-conjugated anti-CD8 were obtained from Immunotech (Marseilelles Cedex, France). FITC-conjugated PE-conjugated anti-CD161, PE-conjugated anti-IL-2Rβ, PE-conjugated anti-HLA-DR and PE-conjugated anti-CD4 were obtained from Becton Dickinson. PE-conjugated anti-CD86 was obtained from Pharmingen (San Diego, CA). PE-conjugated anti-CD57 was obtained from SIGMA (Saint Louis, Missouri) and used as iso-type controls. Cultured cells were harvested and resuspended in 10% fetal calf serum (FCS)-RPMI1620 medium at a density of 1 x 10⁵/ml. After centrifugation for 3 min at 2000 rpm using MRX-150 high speed refrigerated micro centrifuges (TOMY SEIKO CO., LTD. Tokyo, Japan), the cells were resuspended in 50 µl of PBS containing 1 µl of antibodies described above and incubated for 30 min at 4°C. The cells were then washed twice with PBS, resuspended in PBS and stored at 4°C until analysis. Data are shown as FSC, SSC, FL-1 indicating Vα24 or CD4, and FL-2 indicating Vβ11, CD8, CD3.

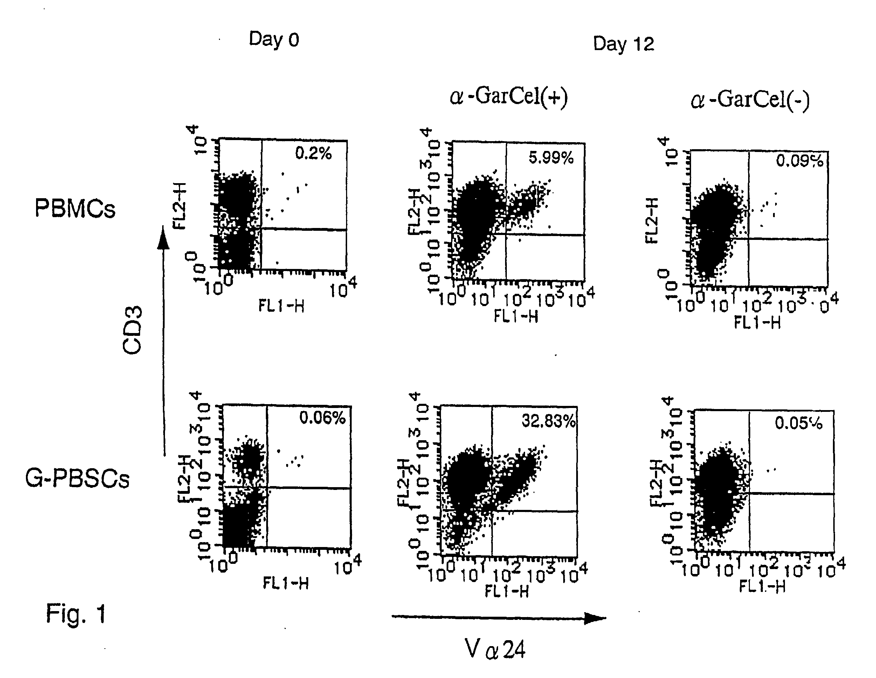

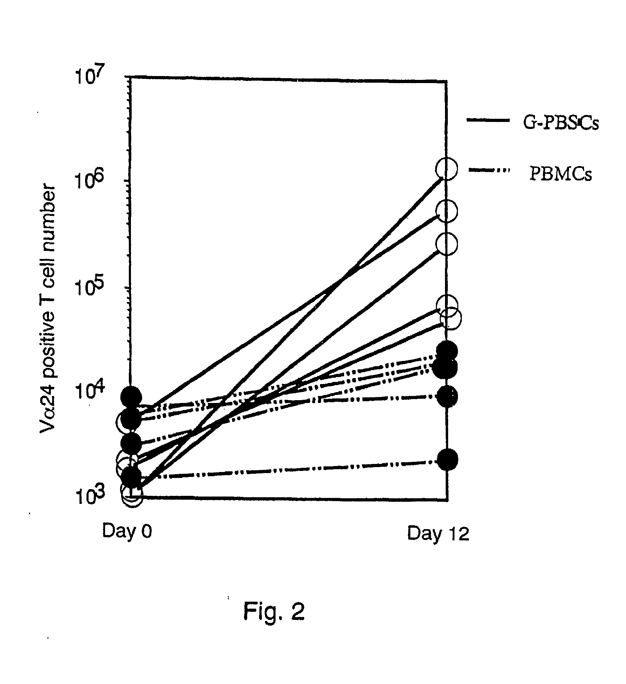

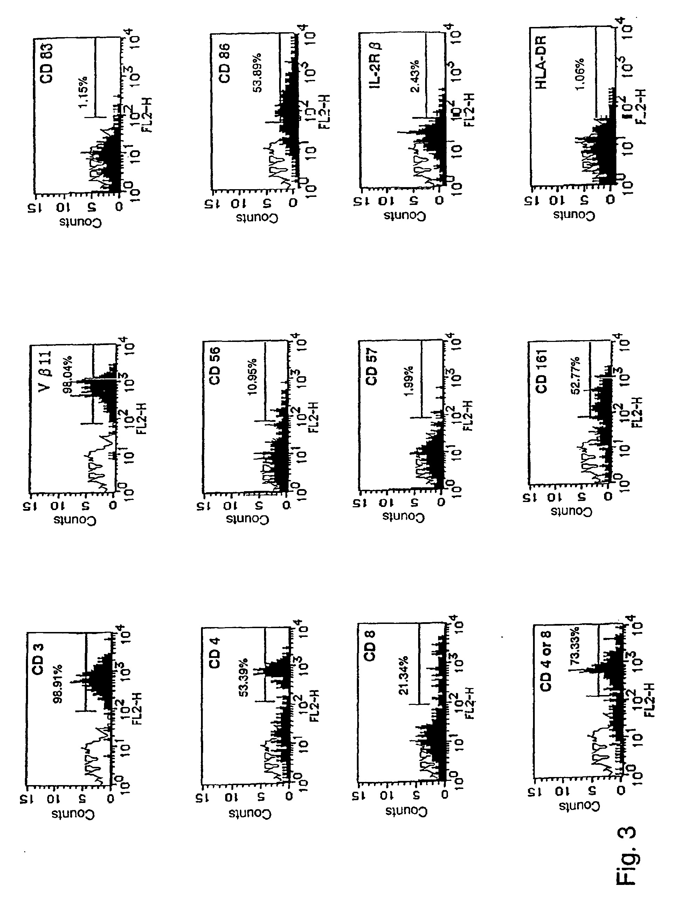

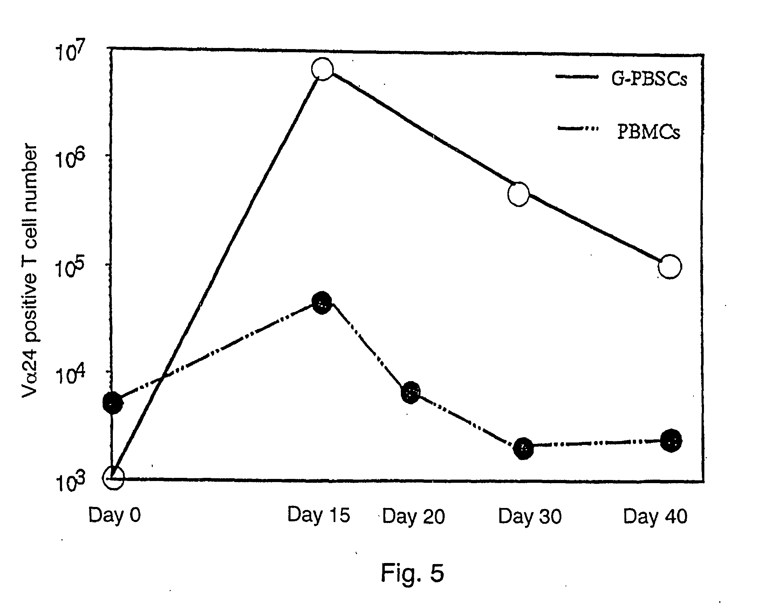

The change of Vα24⁺ NKT cells between day 0 and day 12 is shown in Fig. 1. Vα24⁺ NKT cells from PBMCs expanded in response to IL-2 and α-GalCer. The data from a series of PBMCs from five different donors is shown in Table 2. Whereas Vα24⁺ NKT cells accounted for 0.03-0.14% of the total number of cells on day 0, this percentage increased 1.4 to 6.1 fold by day 12. In the case of G-PBSCs-derived Vα24⁺ NKT cells, amplification on day 12 represented a 32 to 1414-fold increment (Fig. 1, Table 3). This amplification was at least 10-fold higher than that observed in PBMCs (Fig. 2). Figure 3 shows the phenotypes of Vα24⁺ NKT cells on day 12. More than 98% of these cells express TCR Vβ11 and 73.33% of these cells express CD4 or CD8, while less than 5% of Vα24⁺ NKT cells express CD57, CD83, IL-2Rβ, HLA-DR, respectively (Fig. 3). In addition, although the number of Vα24⁺ NKT cells originated from the PBMCs gradually decrease after day 12, those from the G-PBSCs continue to expand up to day 30 of culture (Fig. 5).

**Table 2.**

| Expansion of Vα24⁺ NKT cells from normal PBMCs by α-GalCer | | | | | |
|---|---|---|---|---|---|
| No. | d0 NTK% (cell number) | D12 NKT% (cell number) | | α-GalCer (+)/(-) | d12/d0 |
| | | α-GalCer (+) | α-GalCer (-) | | |
| 1 | 0.12(6000) | 6.58(24346) | 0.03(1575) | 15.5 | 4.1 |
| 2 | 0.14(7000) | 0.19(9500) | 0.26(9152) | 1.0 | 1.4 |
| 3 | 0.06(3000) | 0.3 (18300) | 0.06(4440) | 4.1 | 6.1 |
| 4 | 0.10(5000) | 0.66(20328) | 0.18(4554) | 4.5 | 4.1 |
| 5 | 0.03(1500) | 0.18(2376) | 0.11(1089) | 2.2 | 1.6 |
| d0: day 0, d12: day 12 | | | | | |
| α-GalCer(+): with α-GalCer, α-GalCer(-): without α-GalCer | | | | | |

**Table 3.**

| Expansion of Vα24⁺ NKT cells from G-PBSCs by α-GalCer | | | | | |
|---|---|---|---|---|---|
| No. | d0 NTK% (cell number) | D12 NKT% (cell number) | | α-GalCer (+)/(-) | d12/d0 |
| | | α-GalCer (+) | α-GalCer (-) | | |
| 6 | 0.04(2000) | 3.21(64200) | 0.02(560) | 114.6 | 32.1 |
| 7 | 0.02(1000) | 22.6(1414375) | 0.04(2100) | 673.5 | 1414.4 |
| 8 | 0.03(1500) | 2.05(51660) | 0.01(252) | 205.0 | 34.4 |
| 9 | 0.02(1000) | 8.49(314130) | 0.05(1595) | 196.9 | 314.1 |
| 10 | 0.10(5000) | 4.73(520300) | 0.04(5120) | 101.6 | 104.1 |
| d0: day 0, d12: day 12 | | | | | |
| α-GalCer(+): with α-GalCer, α-GalCer(-): without α-GalCer | | | | | |

It was also determined that G-PBSCs from healthy persons were expanded to the same degree as G-PBSCs of cancer patients.

As is clear from the above results, by using G-PBSCs, at least 10⁸ of Vα24⁺ NKT cells can be produced with a short-term culture.

### Example 2 Evaluation of cytotoxic activity of Vα24⁺ NKT cells against human tumor cells

Human Vα24⁺ NKT cells were purified from the cultured cells on day 12 by FACS Vantage cell sorting system and both their cytotoxic activity against tumor cells and cytokine producing activity were evaluated.

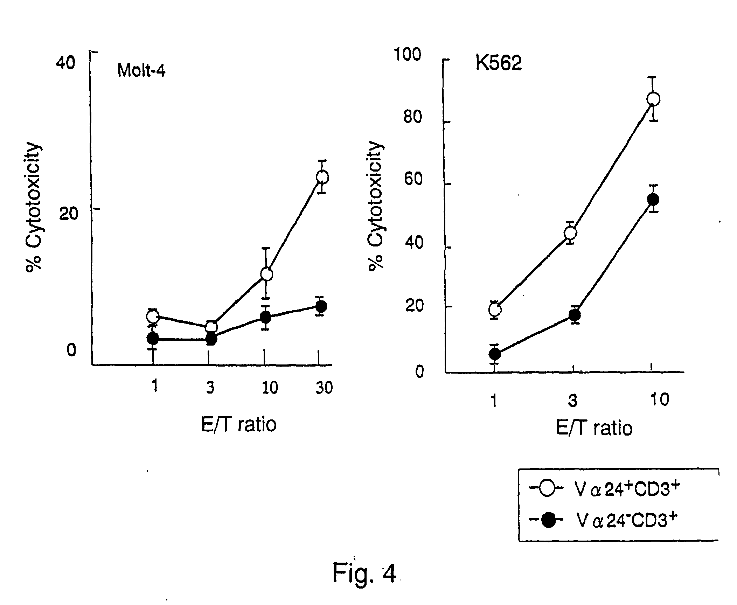

The cytotoxicity of α-GalCer-activated Vα24⁺ NKT cells was determined in triplicate using the following target cell lines; Molt-4 T lymphoma and K562 myelogenous leukemia (ATCC, Pockville, MD). Target cells were labeled with 100 µl Ci sodium chromate (NEN Life Science Products, Inc., Boston MA02118) per 1 x 10⁶ cells for 1 hr. Purified α-GalCer-activated Vα24⁺ NKT cells or Vα24⁻ NKT cells were used as effector cells and seeded onto 96-well round-bottomed plates a t the indicated effector (E)/target (T) ratios on ^{5l}Cr-labeled each target cells. Radioactivity released from lysed target cells was counted using a γ-counter after incubation for 6 hr at 37°C in 5% CO₂. The percentage of specific lysis was calculated from (sample cpm - background cpm)/(maximum cpm - background cpm) x 100. Background cpm was calculated from the supernatant of the target cells alone, and the maximum release was obtained by adding 1 M HCl to target cells. The data are expressed as a mean value of triplicate cultures with standard deviations (Fig. 4).

More than 78% of the cultured cells were found to express the invariant Vα24⁺/Vβ11⁺ TCR. As shown in Fig. 4, these cells displayed potent cytotoxic activity against K562 and Molt-4 tumor cells.

CD14⁺ cells were isolated from mononuclear cells using a magnetic cell sorter. To obtain monocyte-derived DCs, these cells were cultured with RPMI-1640 supplemented with 2 mM L-glutamine, 1% pyruvate, 2% bicarbonate, 100 U/ml penicillin, 100 U/ml streptomycin (GIBCO BRL, Gaithersburg, MD) and 10% FCS (Dainippon Pharmaceutical Co., Ltd.) in the presence of 50 ng/ml recombinant human (rh) GM-CSF, 1000 U/ml rhIL-4 and 500 U/ml rhTNF-α (SIGMA, St Louis, MO). Cells were incubated in a water-vapor-saturated atmosphere containing 5% CO² at 37°C. Sorted Vα24⁺ NKT cells were cultured with monocyte-derived DCs from the same donor during 24 hours. IFN-γ and IL-4 in the culture supernatants were measured using the ELISA system (DIACLONE, BESANCON, FRANCE).

Cytokine secretion from Vα24⁺ NKT cells was measured by ELISA after 48 hr culture in the presence of autologous monocyte-derived DCs. Whereas the secretion of IFN-γ from the Vα24⁺ NKT cells was confirmed, the secretion of IL-4 could not be confirmed.

### Industrial Applicability

According to the present invention, a large amount of Vα24⁺ NKT cells can be produced with a short-term culture. Also, a cell fraction in which Vα24⁺ NKT cells are expanded can be obtained. By using the whole of the fraction, a cell therapy which causes a wider range of immune responses which has not been expected by the conventional cell therapy is expected.

## Claims

1. A method for expanding human Vα24⁺ natural killer T cells, comprising culturing a mononuclear cell fraction obtainable from human peripheral blood in which hemopoietic stem cells are mobilized by granulocyte colony-stimulating factor (G-PBSCs), in the presence of a cytokine effecting proliferation and/or activation of lymphocytes and α-glycosylceramide (α-GlyCer).

2. A method according to claim 1, wherein the cytokine effecting proliferation and/or activation of lymphocytes comprises interleukin 2.

3. A method for producing human Vα24⁺ natural killer T cells, comprising culturing a mononuclear cell fraction obtainable from human peripheral blood in which hemopoietic stem cells are mobilized by granulocyte colony-stimulating factor (G-PBSCs), in the presence of a cytokine effecting proliferation and/or activation of lymphocytes and α-glycosylceramide, and isolating human Vα24⁺ natural killer T cells from the cultured cells.

4. A method according to claim 3, wherein the cytokine effecting proliferation and/or activation of lymphocytes comprises interleukin 2.

5. A method for producing a cell fraction comprising human Vα24⁺ natural killer T cells, comprising culturing a mononuclear cell fraction obtainable from human peripheral blood in which hemopoietic stem cells are mobilized by granulocyte colony-stimulating factor (G-PBSCs), in the presence of a cytokine effecting proliferation and/or activation of lymphocytes and α-glycosylceramide.

6. A method according to claim 5, further comprising culturing cells obtained by the culture in the presence of the cytokine effecting proliferation and/or activation of lymphocytes and α-glycosylceramide, in the presence of a tumor antigen.

7. A method according to claim 5 or 6, wherein the cytokine effecting proliferation and/or activation of lymphocytes comprises interleukin 2.

8. An agent for treating cancer, comprising, as an active ingredient, human Vα24⁺ natural killer T cells obtained by the method as defined in claim 3 or 4.

9. An agent for treating cancer, comprising, as an active ingredient, a cell fraction comprising human Vα24⁺ natural killer T cells obtained by the method as defined in any one of claims 5 to 7.
